# EUROPÄISCHE PATENTSCHRIFT

(11) **EP 1 070 706 B1**
(45) Veröffentlichungstag und Bekanntmachung des Hinweises auf die Patenterteilung: **18.02.2004**
(21) Anmeldenummer: 00114110.0
(22) Anmeldetag: 10.07.2000
(51) Int. Cl.: C07D 233/66

(54) **Verfahren zur Herstellung von 1-substituierten 5-Hydroxy-imidazolin-2,4-dionen und 1-substituierten 5-Alkoxy-imidazolin-2,4-dionen**
Process for the preparation of 1-substituted 5-hydroxy-imidazolin-2,4-diones and 1-substituted 5-alkoxy-imidazolin-2,4-diones
Procédé pour la préparation de 5-hydroxy-imidazolin-2,4-diones substitués dams la position 1 et de 5-alkoxy-imidazolin-2,4-diones substitués dans la position 1

(30) Priorität: 21.07.1999 DE 19934231
(43) Veröffentlichungstag der Anmeldung: 24.01.2001
(73) Patentinhaber: Bayer Chemicals AG, 51368 Leverkusen (DE)
(72) Erfinder: Broda, Witold, Dr., 53819 Neunkirchen-Seelscheid (DE); Vanmaele, Luc Jerome, Dr., 9130 Lochristi (BE)

(56) Entgegenhaltungen:
- EP-A- 0 160 618
- PATENT ABSTRACTS OF JAPAN vol. 011, no. 371 (C-462), 3. Dezember 1987 (1987-12-03) & JP 62 145068 A (NIPPON ZOKI PHARMACEUT CO LTD), 29. Juni 1987 (1987-06-29) & CHEMICAL ABSTRACTS, vol. 107, no. 19, 1987 Columbus, Ohio, US; abstract no. 176040e,
- PATENT ABSTRACTS OF JAPAN vol. 1998, no. 01, 30. Januar 1998 (1998-01-30) & JP 09 227526 A (FUJI PHOTO FILM CO LTD), 2. September 1997 (1997-09-02) & CHEMICAL ABSTRACTS, vol. 127, no. 29, 1997 Columbus, Ohio, US; abstract no. 278194p, Seite 701;
- DOV BEN-ISHAI, ET AL: "The reactions of ureas with glyoxylic acid and methyl glyoxylate" TETRAHEDRON, Bd. 33, 1977, Seiten 1191-1196, XP000940734
- CHEMICAL ABSTRACTS, vol. 119, no. 9, 1993 Columbus, Ohio, US; abstract no. 95415b, Seite 1086; XP002148672 & DING, YU ET AL.: "Synthesis of 1-benzyl-5-ethoxy-2,4-imidazolidinedione" HUAXUE SHIJI, Bd. 15, Nr. 1, 1993, Seiten 15-16,

## Beschreibung

Die Erfindung betrifft ein neues Verfahren zur Herstellung von speziellen 1-substituierten 5-Hydroxy-imidazolin-2,4-dionen ausgehend von N-substituiertem Harnstoff und Glyoxylsäure und die weitere Umsetzung dieser 1-substituierten 5-Hydroxy-imidazolin-2,4-dione zu 1-substituierten 5-Alkoxy-imidazolin-2,4-dionen.

1-Benzyl-5-ethoxy-imidazolin-2,4-dion der Formel (A) 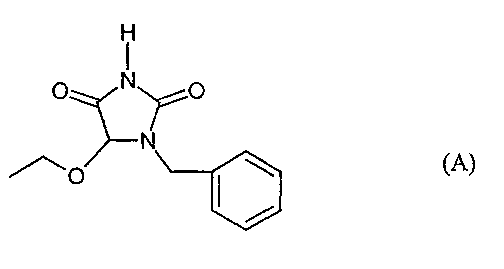 (im weiteren "BEH" genannt) gehört zur Klasse der Hydantoine und wird auch 1-Benzyl-5-ethoxyhydantoin genannt. BEH, seine am Benzylring substituierten Derivate sowie auch andere 1-substituierte 5-Alkoxy-imidazolin-2,4-dione besitzen zunehmende Bedeutung als Zwischenprodukte bei der Herstellung von Arzneimitteln, Insektiziden, Textilhilfsmitteln und Aminosäuren. BEH selbst wird insbesondere für die Herstellung von Photochemikalien benötigt.

Aus Huaxue Shiji 1993, 15(1), 15-16 ist es bekannt, dass BEH aus dem entsprechenden 1-Benzylhydantoin durch Bromierung oder Chlorierung in der 5-Stellung zu den entsprechenden 1-Benzyl-5-halogenhydantoinen und weitere Umsetzung dieser Halogenhydantoine mit Ethanol hergestellt werden kann (siehe nachfolgende Reaktionsgleichung).

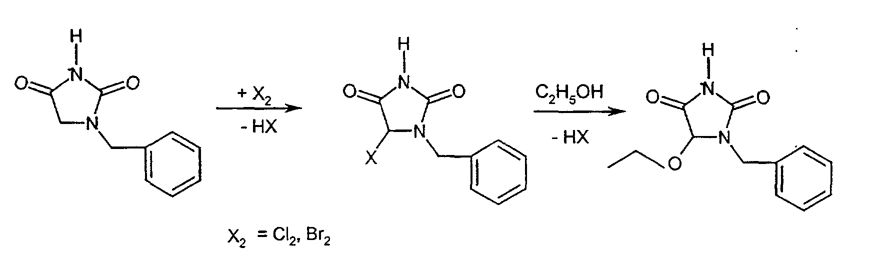

Die zuvor genannte Reaktionsfolge weist mehrere Nachteile auf: So ist die Verwendung von freiem Brom oder Chlor in Bezug auf die großtechnische Handhabung aufwendig und nicht ungefährlich. Ferner fallen bei der Halogenierung selber und auch bei dem anschließenden Halogenaustausch große Mengen an Halogenwasserstoffen an, die entsorgt werden müssen.

Die Ausgangssubstanz der oben genannten Chlorierung oder Bromierung, das 1-Benzylhydantoin, welches auch als 1-Benzyl-imidazolin-2,4-dion bezeichnet wird, muß wiederum über mehrere Schritte hergestellt werden:
a) durch Umsetzung von N-Benzylaminoacetonitril (ein Produkt der Benzylamin- und Blausäureanlagerung an Formaldehyd) und Cyansäure (JP 06 100 543 A2) oder
b) durch Umsetzung von N-Benzylglycin (oder seinen Derivaten) und Harnstoff oder Cyansäure (Huaxue Shiji 1993 15(1), 15-16).

Der Ausgangsstoff des zuvor genannten Syntheseweges a), N-Benzylaminoacetonitril, wird durch Umsetzung von Benzylamin und Formaldehyd mit der extrem giftigen Blausäure hergestellt (siehe auch Tetrahedron Letters [23], 27 (1982), 2741-4). Auch der Ausgangsstoff des Syntheseweges b), N-Benzylglycin, muß erst durch Umsetzung von Glycin mit Benzylchlorid oder von Chloressigsäure mit Benzylamin hergestellt werden. Die Umsetzung von N-Benzylaminoacetonitril bzw. N-Benzylglycin gemäß a) oder b) erfolgt entweder durch langandauerndes Verschmelzen mit Harnstoff oder durch Reaktion mit der giftigen Cyansäure. Beide Methoden liefern 1-Benzyl-hydantoin in nur geringen Ausbeuten: So wird gemäß Huaxue Shiji 1993, 15(1) 15-16 bei der Umsetzung von N-Benzylglycin (gewonnen durch Reaktion von Benzylamin mit Chloressigsäure) mit Cyansäure nur eine Ausbeute von 39.5 % zum 1-Benzyl-hydantoin und bei der Umsetzung von N-Benzylglycin mit Harnstoff nur eine Ausbeute von 45.6 % zum 1-Benzylhydantoin erreicht. Die nachfolgende Bromierung des 1-Benzylhydantoins und Behandlung mit Ethanol verläuft wiederum nur mit einer geringen Ausbeute von 42.7 %.

In Huaxue Shiji 1993 15(1), 15-16 wird ferner lediglich erwähnt, dass man Glyoxylsäure prinzipiell als Ausgangsstoff für eine Umsetzung mit substituiertem Harnstoff verwenden kann. Es werden jedoch keinerlei Angaben dazu gemacht, welche Reaktionsbedingungen zwecks erfolgreicher Durchführung einer solchen Umsetzung eingehalten werden müssen.

Es ist ferner aus JP 09 227 526 A2 bekannt, N-substituierte Harnstoffe der Formel R''NHCONH₂, wobei R"= Alkyl oder Aryl ist, mit Glyoxylsäurealkylester-Alkylhemiacetalen der Formel ROCH(OH)COOR' mit R, R'=Alkyl in einem Lösungsmittel oder einem Lösungsmittelgemisch umzusetzen. Hierbei wird zunächst neben einer großen Zahl anderer Verbindungen das entsprechende 1-Alkyl- oder 1-Arylsubstituierte 5-Hydroxy-hydantoin der Formel (B) gebildet.

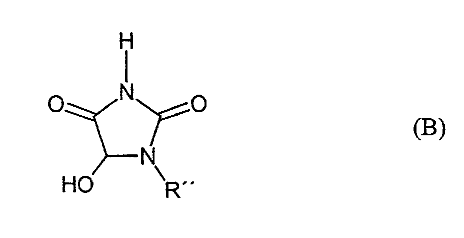

Im Fall von R" = Benzyl entsteht somit 1-Benzyl-5-hydroxyhydantoin der Formel (C) 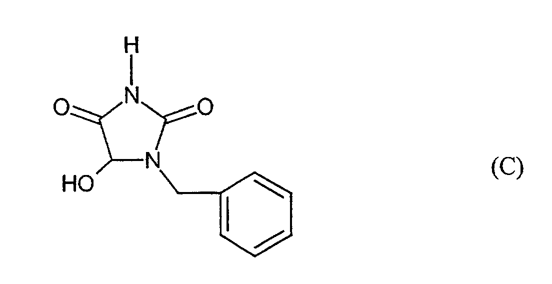 neben vielen anderen Verbindungen. Das Vielstoff-Reaktionsgemisch, welches auch das 1-Alkyl oder 1-Aryl substituierte 5-Hydroxy-hydantoin enthält, liegt als zähe, ölige Masse vor und ist kaum zu reinigen. Vor der weiteren Umsetzung muß es sorgfältig entwässert werden und anschließend durch längeres Erhitzen mit einem Alkohol und Mineralsäure umgesetzt werden, wobei wiederum - aufgrund der unreinen Zusammensetzung des eingesetzten Reaktionsgemisches- ein Gemisch mehrerer Verbindungen gebildet wird, unter anderem auch das gewünschte 1-Alkyl- bzw. 1-Aryl-5-alkoxy-imidazolin-2,4-dion.

Die Isolierung des gewünschten 1-Alkyl- bzw. 1-Aryl-5-alkoxy-imidazolin-2,4-dions ist daher aufwendig und muß durch Säulenchromatographie erfolgen. Diese Trennung ist nur im Gramm-Maßstab beschrieben, in der Technik kaum durchfiihrbar und liefert das gewünschte Produkt mit nur 44 %iger Ausbeute und einer unbekannter Reinheit (JP 09 227 526 A2, Beispiel 1).

Die Ausgangsverbindungen für diese Synthese, d.h. die Glyoxylsäurealkylester-Alkylhemiacetale müssen zudem erst auf unabhängigen Wegen synthetisiert werden. Sie fallen dabei als Gemische von Hemiacetalen und Acetalen an und müssen ebenfalls aufwendig gereinigt werden.

In EP-A-0 160 618 wird die Umsetzung von Glyoxylsäureestern oder O-Alkylglyoxylsäureestem (Glyoxylsäureesteralkoholaten) mit N-Alkylharnstoffen, N-Cycloalkylharnstoffen, N,N'-Dialkylharnstoffen oder N,N'-Dicycloalkylharnstoffen in einem Lösungmittel wie beispielsweise Wasser und/oder Essigsäure beschrieben. Es wird darüberhinaus festgestellt, dass diese Umsetzung auch mit Glyoxylsäure selber durchgeführt werden könne. In Beispiel 2 von EP-A-0 160 618 wird die Umsetzung von Glyoxylsäure mit N-Methylharnstoff in einer wässrigen Essigsäurelösung durchgeführt. Als Produkt wird 5-Hydroxy-3-methylhydantoin angegeben, es fehlen jedoch jegliche Angaben zur Ausbeute oder Selektivität der Reaktion. Eine Nacharbeitung der prinzipiellen Umsetzung von Glyoxylsäure mit N-Methylharnstoff lieferte nur geringe Mengen schmierigen Kristallisats, welches ein sehr kompliziertes Gemisch mehrerer Substanzen darstellt und das durch Kristallisation nicht vernünftig getrennt werden kann. Eine NMR-Analyse dieses Kristallisats ergab neben vielen anderen Verbindungen in geringen Mengen auch die beiden isomeren 1- und 3-Methyl-5-hydroxyhydantoine in etwa gleichen Mengen von ca. 10%. Im Hinblick auf die fehlende Ausbeuteangabe in Beispiel 2 der EP-A- 0 160 618 ist daher davon auszugehen, dass dort nur die kleinen Mengen des 1-Methyl-5-hydroxy-hydantoins isoliert wurden. Das Verfahren der EP-A-0 160 618 unter Einsatz Alkyl- oder Cycloalkyl-substituierter Harnstoffe kann somit nicht als geeignete Möglichkeit zur Darstellung von 1-Alkyl-5-hydroxy- hydantoinen erachtet werden.

Auch aus Tetrahedron 33 (1977), S 1191-1196 ist die Umsetzung von Glyoxylsäure mit N-Methylharnstoff bekannt. Ohne Verwendung eines Katalysators liefert diese Umsetzung in Methanol allerdings 5-Methoxy-3-methylhydantoin.

Da der Bedarf an 1-substituierten 5-Alkoxy-imidazolin-2,4-dionen im Hinblick auf die bereits genannte Vielzahl von Anwendungsmöglichkeiten stetig zunimmt, bestand die Aufgabe der vorliegenden Erfindung darin, ein Verfahren bereitzustellen, mit dem die 1-substituierten 5-Hydroxy-imidazolin-2,4-dione als Zwischenprodukte zur Synthese der 1-substituierten 5-Alkoxy-imidazolin-2,4-dione, unter Einsatz einfach zu handhabender und nicht giftiger Chemikalien mit hoher Ausbeute und hoher Reinheit hergestellt werden können. Insbesondere soll das bereitzustellende Verfahren eine aufwendige Reinigung der 1-substituierten 5-Hydroxy-imidazolin-2,4-dione, z.B. über Säulenchromatographie, überflüssig machen.

Gelöst wird diese Aufgabe durch ein Verfahren zur Herstellung von 1-substituierten 5-Hydroxy-imidazolin-2,4-dionen der Formel (I) 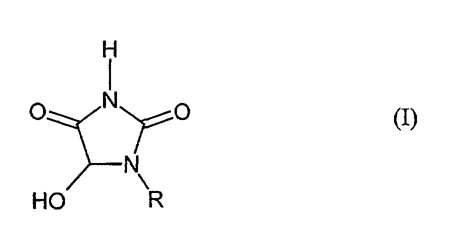 wobei R für einen gegebenenfalls substituierten C₇-C₁₈-Aralkylrest steht, durch Umsetzung von Glyoxylsäure mit N-substituiertem Harnstoff der Formel RNH-CO-NH₂, wobei R die zuvor genannte Bedeutung besitzt, dadurch gekennzeichnet, dass das Verfahren in einer 10-80 %igen wäßrigen Lösung in Gegenwart eines sauren Katalysators durchgeführt wird.

Überraschenderweise kann die Umsetzung von Glyoxylsäure und dem N-substituierten Harnstoff in wäßriger Lösung und in Gegenwart des sauren Katalysators erfolgreich und kontrolliert durchgeführt werden. Wesentlich ist hierbei, dass die Durchführung in wäßriger Lösung erfolgt. Dies hat zur Folge, dass die Glyoxylsäure überwiegend in Form des Hydrats vorliegt.

Das erfindungsgemäße Verfahren zeichnet sich durch eine hohe Selektivität zum 1-substituierten 5-Hydroxy-imidazolin-2,4-dion aus, die mindestens bei 60 %, überwiegend bei mindestens 70 % und oft sogar bei 75 % oder mehr liegt. Neben dem 1-substituierten 5-Hydroxy-imidazolin-2,4-dion werden die gemäß der nachfolgenden Reaktionsgleichung möglichen Nebenprodukte nur in sehr geringen Mengen gebildet.

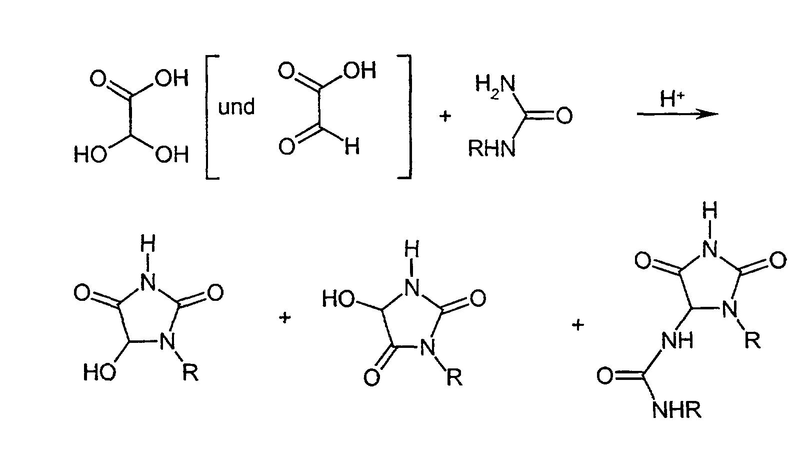

Die im erfindungsgemäßen Verfahren eingesetzten N-substituierten Harnstoffe besitzen die Formel RNH-CO-NH₂, wobei R für einen gegebenenfalls substituierten C₇-C₁₈-Aralkylrest steht. Die Arylreste können dabei durch 1, 2, 3, 4 oder 5 gleiche oder verschiedene Reste aus der Gruppe der Halogen-, bevorzugt Chlor, C₁-C₁₂-Alkyl-, bevorzugt Methyl, NO₂-, C₁-C₁₂-Alkoxy-, bevorzugt Methoxy, und Phenoxyreste substituiert sein.

Bevorzugt werden N-substituierte Harnstoffe der Formel RNH-CO-NH₂ eingesetzt, bei denen R für einen gegebenenfalls substituierten C₇-C₁₂-Aralkylrest steht. Besonders bevorzugt steht R für einen Benzylrest oder Benzylreste, die durch 1, 2, 3, 4 oder 5 der obengenannten Reste substituiert sind, wie beispielsweise 3,4-Dimethoxybenzyl, 4-Methylbenzyl oder 4-Chlorbenzyl. Als Aralkylreste sind ferner 3-Phenylpropyl, 1-Phenylethyl und 2- Phenylethyl geeignet. Als substituierter Arylrest ist ferner 4-Chlorphenyl geeignet.

Das erfindungsgemäße Verfahren wird in Anwesenheit eines sauren Katalysators durchgeführt. Besonders bewährt hat sich als Katalysator Essigsäure. Einsetzbar sind auch solche Katalysatoren, deren pKₐ-Werte dem der Essigsäure ähnlich sind. Dazu gehören Kalium- oder Natriumdihydrogenphosphate oder Kalium- oder Natriumhydrogenphosphate. Ebenfalls einsetzbar sind auch andere saure Katalysatoren wie z.B. Ameisensäure, Propionsäure, Borsäure, Phosphorsäure, Oxalsäure oder Alkalimetallhydrogensulfate.

Das molare Verhältnis der Glyoxylsäure zum N-substituierten Harnstoff beträgt (0,5-2):1, bevorzugt (0,8-1,2):1. Besonders bevorzugt ist die Verwendung äquimolarer (oder fast äquimolarer) Mengen an Glyoxylsäure und N-substituiertem Harnstoff.

Wesentlich für das erfindungsgemäße Verfahren ist die Durchführung in einer 10-80 %igen, bevorzugt 20-70 %igen und insbesondere 40-60 %igen wäßrigen Lösung. Die Glyoxylsäure kann entsprechend z.B. in Form ihrer ungefähr 50 %igen wäßrigen Lösung eingesetzt werden.

Im erfindungsgemäßen Verfahren können darüber hinaus auch noch andere organische Lösungsmittel zugegen sein wie z.B. Kohlenwasserstoffe, Alkohole oder Ester.

Das erfindungsgemäße Verfahren wird üblicherweise bei einer Temperatur im Bereich von 80-120°, bevorzugt 95-105°C durchgeführt. Bei Reaktionstemperaturen nahe dem Siedepunkt des Reaktionsgemisches (um 100°C) ist die Umsetzung zwischen Glyoxylsäure und dem N-substituierten Harnstoff schnell und vollständig. Um Restgehalte an N-substituiertem Harnstoff unter 1 % zu erreichen, hat es sich bewährt, das Reaktionsgemisch nach der eigentlichen Umsetzung für 1-2 Stunden nachzurühren.

Besonders bewährt hat sich das erfindungsgemäße Verfahren zur Herstellung von 1-Benzyl-5-hydroxy-imidazolin-2,4-dion durch Umsetzung von Benzylharnstoff mit Glyoxylsäure in wäßriger Lösung in Gegenwart eines sauren Katalysators, insbesondere Essigsäure.

Die Isolierung des gewünschten Produktes, des 1-substituierten 5-Hydroxy-imidazolin-2,4-dions der Formel (I), erfolgt üblicherweise durch Kristallisation, die entweder mit oder ohne zusätzliche Zugabe von Lösungsmittel durchgeführt werden kann. Bevorzugt wird dem Kristallisationsgemisch zusätzlich ein Lösungmittel zugegeben, da sich dann ein Kristallisat höherer Reinheit bildet, das sich sehr gut abfiltrieren läßt. Als Lösungsmittel geeignet sind allgemein aliphatische Kohlenwasserstoffe wie Hexan, Heptan oder Isooctan, halogenierte Kohlenwasserstoffe wie Methylenchlorid, gegebenenfalls substituierte aromatische Kohlenwasserstoffe wie Benzol, Toluol, Ethylbenzol sowie chlorierte Benzole oder Toluole, Ketone wie Aceton oder Methylethylketon oder Ether wie Methyl-tert.butylether oder Methylisopropylether. Besonders bewährt hat sich die Zugabe von Methylenchlorid oder Chlorbenzol. Die Kristallisationstemperatur liegt bevorzugt unter 40°C. Wenn bei der Kristallisation keine zusätzlichen Lösungsmittel verwendet werden, so kristallisiert das Produkt zunächst noch zusammen mit einer kleineren Menge an Verunreinigungen aus. Insbesondere in diesem Fall hat es sich bewährt, das gewünschte Produkt, das 1-substituierte 5-Hydroxy-imidazolin-2,4-dion, auf einfache Weise durch Umkristallisation zu reinigen. Bei zu hohen Gehalten an 5-Benzylharnstoff-1-benzyl-imidazolin-2,4-dion hat sich die Kristallisation aus heißem Wasser als sehr vorteilhaft herausgestellt. Die Löslichkeit dieses Nebenproduktes in heißem Wasser ist gering, und so kann es aus der heißen Lösung des gewünschten 1-substituierten 5-Hydroxy-imidazolin-2,4-dions abfiltriert werden.

Vor einer weiteren Umsetzung des isolierten 1-substituierten 5-Hydroxy-imidazolin-2,4-dions ist eine weitergehende Trocknung des 1-substituierten 5-Hydroxy-imidazolin-2,4-dions nicht unbedingt notwendig. Es hat sich aber herausgestellt, dass die Chemikalienverluste in der weiteren Umsetzung zum 1-substituierten 5-Alkoxyimidazolin-2,4-dion minimiert werden können, wenn das 1-substituierte 5-Hydroxyimidazolin-2,4-dion möglichst wenig Wasser enthält. Es kann daher gewünschtenfalls durch einfache Luft- oder Vakuumtrocknung bei Raumtemperatur oder erhöhter Temperatur oder auch durch azeotrope Wasserdestillation mit Hilfe geeigneter Wasserschleppmittel getrocknet werden.

Das Reaktionsprodukt der Umsetzung zwischen Glyoxylsäure und dem N-substituierten Harnstoff, insbesondere das bevorzugte 1-Benzyl-5-hydroxy-imidazolin-2,4-dion, muß aber nicht unbedingt zwischenisoliert werden. Es ist auch möglich, aus dem rohen Reaktionsgemisch den größten Teil des Lösungsmittels durch Destillation zu entfernen und das ölige Produkt in dieser Form unmittelbar weiter umzusetzen. Des weiteren ist es möglich, durch Abkühlen des rohen Reaktionsgemisches das Rohprodukt als ölige Unterphase abzutrennen.

In einer weiteren Ausgestaltung des erfindungsgemäßen Verfahrens werden die hergestellten 1-substituierten 5-Hydroxy-imidazolin-2,4-dione der Formel I in einem weiteren Schritt zu 1-substituierten 5-Alkoxy-imidazolin-2,4-dionen der Formel II 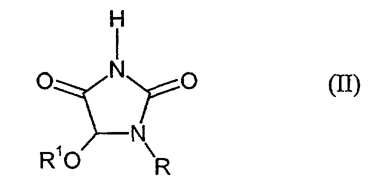 umgesetzt, wobei R die für die Formel I genannte Bedeutung hat und R¹ für einen geradkettigen oder verzweigten C₁-C₁₈-Alkylrest, bevorzugt für Methyl, Ethyl, Propyl, iso-Propyl, Butyl, iso-Butyl, Octyl oder 2-Ethylhexyl und insbesondere für Ethyl steht.

Die Umsetzung der 1-substituierten 5-Hydroxy-imidazolin-2,4-dione der Formel I zu den 1-substituierten 5-Alkoxy-imidazolin-2,4-dionen der Formel II kann auf mehreren Wegen erfolgen. Geeignet ist z.B.
1) die Umsetzung des 1-substituierten 5-Hydroxy-imidazolin-2,4-dions der Formel I mit einem Alkohol der Formel R¹OH in Gegenwart eines sauren Katalysators.
   Eine solche sauerkatalysierte Veretherung ist beispielsweise in JP 09 227 526 A2 beschrieben. Als Alkohol wird bevorzugt Ethanol verwendet. Das vorzugsweise zu einem Feststoff getrocknete 1-substituierte 5-Hydroxy-imidazolin-2,4-dion, welches insbesondere praktisch wasserfrei ist, wird mit dem Alkohol umgesetzt. Es hat sich bewährt, mindestens 1 mol Alkohol pro mol 1-substituiertem 5-Hydroxy-imidazolin-2,4-dion einzusetzen. Als saurer Katalysator wird vorzugsweise eine Protonensäure eingesetzt. Diese Protonensäure besitzt insbesondere einen negativen pKₐ-Wert. Solche Protonensäuren sind in "Advanced Organic Chemistry" (Hrsg. J. March, John Wiley & Sons 1985, 3. Auflage, Kapitel 8) angegeben. Beispiele sind Salzsäure (pKₐ=-7), Bromwasserstoffsäure (pKₐ=-9), Schwefelsäure (pKₐ=-9) und organische Sulfonsäuren (pKₐ=-6.5). Bevorzugte Beispiele organischer Sulfonsäuren schließen Methansulfonsäure, Trifluormethansulfonsäure, Benzolsulfonsäure und p-Toluolsulfonsäure ein. Die Säure wird in einem Verhältnis von 0.001-10 mol, bevorzugt 0.01-1 mol und insbesondere 0.01-0.03 mol pro mol 1-substituiertem 5-Hydroxy-imidazolin-2,4-dion eingesetzt. Die Reaktionstemperatur liegt bei 0 -120°C, bevorzugt 20-100°C. Die Reaktionszeit beträgt üblicherweise 4-20 Stunden.
2) die Umsetzung des 1-substituierten 5-Hydroxy-imidazolin-2,4-dions der Formel I mit Orthoameisensäuretri-C₁-C₁₈- alkylester, insbesondere Orthoameisensäuretriethylester.
   Diese Umsetzung ist ebenfalls sauer katalysiert und verläuft sehr schnell. Die Reaktionstemperatur liegt im Bereich von 20-160°C, bevorzugt 50-130°C und besonders bevorzugt 70-120°C. Die Reaktionszeit beträgt üblicherweise 1-10 Stunden. Als Katalysatoren können alle Säuren eingesetzt werden, die für die Variante 1) oben aufgeführt wurden. Das molare Verhältnis des Orthoameisensäuretrialkylesters zum 1-substituierten 5-Hydroxy-imidazolin-2,4-dion liegt üblicherweise bei (0.5-5):1. Besonders bevorzugt werden äquimolare Mengen an Orthoameisensäuretrialkylester und 1-substituiertem 5-Hydroxy-imidazolin-2,4-dion eingesetzt.
3) die Umsetzung des 1-substituierten 5-Hydroxy-imidazolin-2,4-dions der Formel I zunächst mit Thionylchlorid und anschließend mit einem Alkohol R¹OH, wobei R¹ die zuvor genannte Bedeutung hat.
   Die Reaktionstemperatur liegt im Bereich von 50-150°C, bevorzugt 60-100°C. Die Reaktionszeit beträgt 1-20 Stunden. Das molare Verhältnis von Thionylchlorid zum 1-substituierten 5-Hydroxy-imidazolin-2,4-dion beträgt (0,5-5):1. Diese Umsetzung kann in Abwesenheit oder Gegenwart eines organischen Lösungsmittels durchgeführt werden. Geeignet sind als Lösungsmittel aliphatische, chlorierte oder aromatische Kohlenwasserstoffe, vorzugsweise Benzol oder Toluol.

Die Isolierung des 1-substituierten 5-Alkoxy-imidazolin-2,4-dions erfolgt bevorzugt durch Kristallisation aus dessen alkoholischen Lösungen, da dies die technisch am einfachsten durchzuführende Operation ist.

Es ist auch möglich, das Produkt selbst als Schmelze herzustellen und diese durch Erkalten erstarren zu lassen oder die Lösungsmittelkristallisation statt aus Ethanol aus anderen Lösungsmittel durchzuführen.

Weitere aufwendige Reinigungsschritte sind nicht notwendig, da das auf allen drei Wegen 1), 2) und 3) erhaltene 1-substituierte 5-Alkoxy-imidazolin-2,4-dion in einer deutlich höheren Reinheit vorliegt als vergleichbare 1-substituierte 5-Alkoxyimidazolin-2,4-dione, die nach den Reaktionsfolgen des Standes der Technik hergestellt werden. Dies ist eine Folge der exzellenten Selektivität des erfindungsgemäßen Verfahrens zum Zwischenprodukt, dem 1-substituierten 5-Hydroxy-imidazolin-2,4-dion.

### Beispiel 1

### Herstellung von 1-Benzyl-5-hydroxy-imidazolin-2,4-dion mit verschiedenen sauren Katalysatoren

In einer beheizbaren Rührapparatur werden 75 Gew.-Teile einer 50 %igen, wäßrigen Glyoxylsäure (0,5 mol Glyoxylsäure) und der Katalysator vorgelegt. Anschließend wird das Gemisch unter Rühren auf 100°C Innentemperatur erhitzt. Dann werden im Verlauf von 30 Minuten mit konstanter Geschwindigkeit 75 Gew.-Teile Benzylharnstoff (entspricht 0,5 mol) hinzugefügt. Nach beendeter Zugabe des Benzylharnstoffs wird das Reaktionsgemisch noch 30 Minuten bei 100°C nachgerührt, eine Probe entnommen und über HPLC analysiert. Die Tabelle 1 zeigt die Zusammensetzung der organischen Anteile der untersuchten Reaktionsgemische. Die nachfolgende Reaktionsgleichung zeigt die Formeln der Verbindungen, die entstehen können.

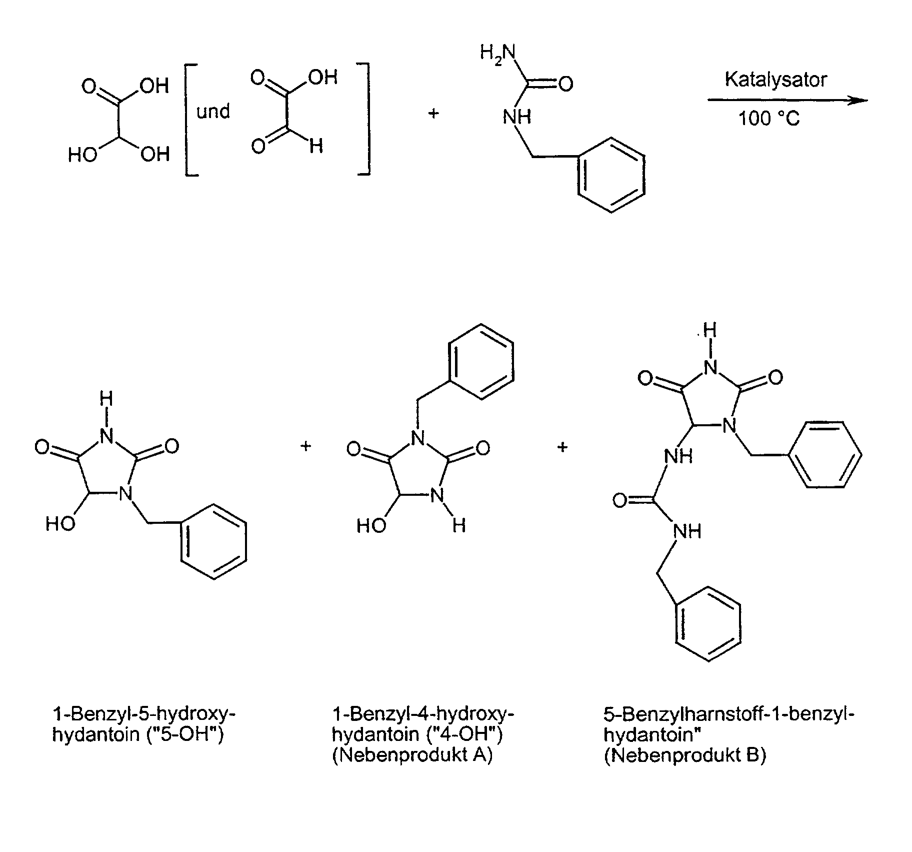

| (bei allen nachfolgenden Angaben in Teilen handelt es sich um Gew.Teile) | | | | |
|---|---|---|---|---|
| Katalysator | Benzylharnstoff | "5-OH" | Nebenprodukt "A" | Nebenprodukt " "B" |
| | Gew.-% | Gew.-% | Gew.-% | Gew.-% |
| 75 Teile Essigsäure | 1,0 | 76,2 | 15,8 | 7,0 |
| 75 Teile Ameisensäure | 0,7 | 67,5 | 14,7 | 17,1 |
| 10 Teile Borsäure gelöst in 75 Teilen H₂O | 0,3 | 78,7 | 9,7 | 11,4 |
| 10 Teile Phosphorsäure gelöst in 75 Teilen H₂O | 0,8 | 66,7 | 17,6 | 14,9 |
| 10 Teile Oxalsäure gelöst in 75 Teilen H₂O | 1,8 | 63,9 | 21,9 | 12,5 |
| 10 Teile Na₂HPO₄ gelöst in 75 Teilen H₂O | 7,0 | 69,0 | 9,1 | 14,9 |
| 10 Teile KH₂PO₄ gelöst in 75 Teilen H₂O | 3,6 | 73,7 | 10,7 | 12,0 |
| 10 Teile KHSO₄ gelöst in 75 Teilen H₂O | 1,4 | 65,6 | 20,2 | 12,9 |
| 10 Teile CH₃COONa gelöst in 75 Teilen H₂O | 16,4 | 65,7 | 3,3 | 14,6 |

### Beispiel 2

### Herstellung von 1-Benzyl-5-hydroxy-imidazolin-2,4-dion

In einer beheizbaren Rührapparatur werden 3000 Gew. -Teile 50 %iger wäßriger Glyoxylsäure-Lösung und 500 Gew.-Teile Essigsäure (100 %ig) vorgelegt und auf 100°C erhitzt. Zu dieser Lösung wird innerhalb von einer Stunde ein 80°C warmes Gemisch aus 3000 Gew.-Teilen Benzylharnstoff, 1300 Gew.-Teilen Wasser und 1500 Gew.-Teilen Essigsäure hinzugefügt. Anschließend wird das gesamte Reaktionsgemisch eine weitere Stunde bei 100°C Innentemperatur gerührt. Dann werden bis zu einem Druck von 200 mbar und einer Sumpftemperatur von 100°C 4500 Gew.-Teile eines Essigsäure/Wasser-Gemisches abdestilliert. Der verbleibende flüssige Rückstand wird bei 90°C mit 900 Gew.-Teilen Wasser und 12,5 Gew.-Teilen Essigsäure versetzt, auf 35°C abgekühlt, mit 3000 Gew.-Teilen Methylenchlorid versetzt und 12 h bei 25°C gerührt. Danach wird durch Kühlung die Temperatur 1 h lang auf 3°C gehalten.

Der resultierende Kristallbrei wird abfilitriert, das Kristallisat mit 1500 Gew.-Teilen Wasser und 2500 Gew.-Teilen Methylenchlorid gewaschen und durch Durchziehen von Stickstoff bis zu Gewichtskonstanz getrocknet.

Es resultieren 2281 Gew.-Teile eines weißen Feststoffs folgender Zusammensetzung:

| | Gew.-% |
|---|---|
| Benzylharnstoff | 0 |
| "5-OH" | 97,2 |
| Nebenprodukt A | 2,8 |
| Nebenprodukt B | 0 |
| Wasser | 0,05 |

### Beispiel 3

### Herstellung von 1-Benzyl-5-ethoxy-imidazolin-2,4-dion unter Verwendung von Orthoameisensäuretriethylester ohne Zwischenisolierung des 1-Benzyl-5-hydroxy-imidazolin-2,4-dions

In einer beheizbaren Rührapparatur werden 3000 Gew.-Teile 50 %iger wäßriger Glyoxylsäure-Lösung und 500 Gew.-Teile Essigsäure vorgelegt und unter Rühren auf 100°C Innentemperatur erhitzt. Zu dieser Lösung wird ein 80°C heißes Gemisch aus 3000 Gew.-Teilen Benzylharnstoff und 2000 Gew.-Teilen Essigsäure im Verlauf von 1 h hinzugefügt. Danach wird das Reaktionsgemisch 2 h bei 100°C nachgerührt. Nach dieser Zeit werden innerhalb von 2 h bei einer Temperatur von 100-110°C bis zu einem Druck von 300 mbar 4000 Gew.-Teile eines Essigsäure/Wasser-Gemisches abdestilliert. Der Rückstand dieser Destillation wird bei 80°C mit 2000 Gew.-Teilen Toluol versetzt und bei 100°C Sumpftemperatur und einem Druck von 300 mbar destilliert. Dem Rückstand werden dann 1000 Gew.-Teile Ethanol, 3000 Gew.-Teile Orthoameisensäuretriethylester und 50 Gew.-Teile H₂SO₄ zugesetzt und eine Stunde auf 80°C erhitzt. In dieser Zeit werden 100 Gew.-Teile Lösungsmittelgemisch - vorwiegend Ethylformiat - abdestilliert. Dem Rückstand wird anschließend ein Gemisch aus 3000 Gew.-Teilen Ethylacetat und 6000 Gew.-Teilen n-Hexan zugesetzt und die resultierende Lösung wird danach 8 h bei 25°C und 3 h bei 3°C gerührt. Der gebildete kristalline Niederschlag wird abfiltriert und bis zu Gewichtskonstanz getrocknet.

Es werden 2000 Gew.-Teile 94 %iges 1-Benzyl-5-ethoxy-imidazolin-2,4-dion erhalten.

### Beispiel 4

### Herstellung von 1-Benzyl-5-ethoxy-imidazolin-2,4-dion aus isoliertem 1-Benzyl- 5-hydroxy-imidazolin-2,4-dion unter Verwendung von Thionylchlorid und Ethanol

In einer beheizbaren Rührapparatur, die an einen Gaswäscher angeschlossen ist, werden 309 Gew.-Teile 1-Benzyl-5-hydroxy-imidazolin-2,4-dion aus Beispiel 2 und 375 Gew.-Teile Toluol vorgelegt. Das Reaktionsgemisch wird zunächst kurz bis zum Sieden erhitzt, um restliche Spuren von Wasser zu entfernen. Danach kühlt man das Reaktionsgemisch auf 90°C ab und gibt bei dieser Temperatur im Verlauf von 4 h unter Rühren 196,5 Gew.-Teile Thionylchlorid hinzu. Aus dem Reaktionsgefäß entweicht ein konstanter, starker Gasstrom (HCl und SO₂). Nach 4 Stunden liegt das Reaktionsgemisch als helle, gelbliche Lösung vor, und die Gasbildung endet. Anschließend wird bei einer Temperatur von 90°C und einem Druck von 200 mbar das Toluol abdestilliert. Die Innentemperatur im Reaktionsgefäß wird nun auf 80°C abgesenkt, und es werden im Verlauf von 3 h 450 Gew.-Teile Ethanol hinzugefügt. Anschließend läßt man 2 h bei einer Temperatur von 82°C nachrühren. Der klaren, hellen Reaktionslösung werden dann 7 Gew.-Teile Aktivkohle zugegeben und 15 Min. lang verrührt. Anschließend wird die Aktivkohle an einem Klärfilter abgetrennt und das Filtrat unter Rühren auf 3°C abgekühlt. Der resultierende Kristallbrei wird abfiltriert und bis zur Gewichtskonstanz getrocknet.

Es werden 278,3 Gew.Teile Produkt enthalten, das laut Analyse 98,2 % 1-Benzyl-5-ethoxy-imidazolin-2,4-dion und 1,8 % Ethanol enthält.

### Beispiele 5(1)-5(7)

### Herstellung weiterer 1-substituierter 5-Hydroxy-imidazolin-2,4-dione

In einer Apparatur, wie sie in Beispiel 2 beschrieben ist, werden in identischer Weise wie in Beispiel 2 durch Umsetzung der unten angegebenen Mengen des N-substituierten Harnstoffs weitere 1-substituierte 5-Hydroxy-imidazolin-2,4-dione mit folgenden Eigenschaften hergestellt:

### Beispiel 5(1)

### 5-Hydroxy-1-(3-phenylpropyl)- imidazolin-2,4-dion, C₁₂H₁₄N₂O₃ wird hergestellt unter Einsatz von 3560 Gew.-Teilen 3-Phenylpropylharnstoff.

Fp.: 131-132°C, MS: Molekularpeak 234, NMR (400MHz; DMSO D₆): 1,7-1,9 (m, 2H); 2,6 (t, 2H); 3,25 (m, 2H); 5,1 (d, I=11Hz, 1H); 6,85 (d, I=11Hz, 1H, austauschbar gegen D); 7,15-7,30 (m, 5H); 10,75 (s, 1H, austauschbar gegen D)

### Beispiel 5(2)

### 5-Hydroxy-1-(3,4-dimethoxybenzyl)-imidazolin-2,4-dion, C₁₂H₁₄N₂O₅ wird hergestellt unter Einsatz von 4260 Gew.-Teilen n-Veratrylharnstoff.

Fp.: 143-144°C, MS: Molekularpeak 266, NMR (400MHz; DMSO D₆): 3,75 (s, 6H); 4,3 (dd, I₁=83Hz, I₂=17Hz, 2H); 4,95 (d, I=10Hz, 1H); 6,85-6,95 (m, 3H); 7,0 (d, I=10Hz, 1H, austauschbar gegen D); 10,85 (s, 1H, austauschbar gegen D)

### Beispiel 5(3)

### 5-Hydroxy-1-(1-phenylethyl)-imidazolin-2,4-dion, C₁₁H₁₂N₂O₃ wird hergestellt unter Einsatz von 3330 Gew.-Teilen DL-1-Phenylethylharnstoff.

Fp.: 139-140°C, MS: Molekularpeak 220, NMR (400MHz; DMSO D₆): 1,55-1,65 (t, 3H); 4,85 (d, I=9Hz, 1H); 5,0-5,1 (q, 1H); 6,9 (d, I=9Hz, 1H, austauschbar gegen D); 7,25-7,45 (m, 5H); 10,8 (s, 1H, austauschbar gegen D)

### Beispiel 5(4)

### 5-Hydroxy-1-(4-methylbenzyl)-imidazolin-2,4-dion, C₁₁H₁₂N₂O₃ wird hergestellt unter Einsatz von 3030 Gew.-Teilen 4-Methylbenzylharnstoff.

Fp.: 174°C, MS: Molekularpeak 220, NMR (400MHz; DMSO D₆): 2,3 (s, 3H); 4,35 (dd, I₁=90Hz, I₂=12Hz, 2H); 4,9 (d, I=9Hz, 1H); 7,0 (d, I=9Hz, 1H, austauschbar gegen D); 7,1-7,2 (m, 4H); 10,9 (s, 1H, austauschbar gegen D)

### Beispiel 5(5)

### 5-Hydroxy-1-(4-chlorobenzyl)-imidazolin-2,4-dion, C₁₀H₉ClN₂O₃ wird hergestellt unter Einsatz von 3120 Gew.-Teilen 4-Chlorbenzylharnstoff.

Fp: 149-150°C, MS: Molekularpeak 240, NMR (400MHz; DMSO D₆): 4,4 (dd, I₁=55Hz, I₂=15Hz, 2H); 5,0 (d, I=10Hz, 1H); 7,0 (d, I=10Hz, 1H, austauschbar gegen D); 7,3-7,4 (m, 4H); 10,9 (s, 1H, austauschbar gegen D)

### Beispiel 5(6)

### 5-Hydroxy-1-[2-(4-chlorophenyl)ethyl]-imidazolin-2,4-dion, C₁₁H₁₁ClN₂O₃ wird hergestellt unter Einsatz von 3350 Gew.-Teilen 4-Chlorethylphenylharnstoff.

Fp.: 151-152°C, MS: Molekularpeak 254, NMR (400MHz; DMSO D₆): 2,75-2,9 (m, 2H); 3,3-3,6 (m, 2H); 5,05 (d, I=11Hz, 1H); 6,95 (d, I=11Hz, 1H, austauschbar gegen D); 7,25-7,40 (m, 4H); 10,75 (s, 1H, austauschbar gegen D)

### Beispiel 5(7)

### 5-Hydroxy-1-(2-Phenylethyl)-imidazolin-2,4-dion, C₁₁H₁₂N₂O₃ wird hergestellt unter Einsatz von 3320 Gew.-Teilen 2-Phenylethylharnstoff.

Fp.: 168-169°C, MS: Molekularpeak 220, NMR (400MHz; DMSO D₆): 2,75-2,9 (m, 2H); 3,3-3,6 (m, 2H); 5,0 (d, I=10Hz, 1H); 6,95 (d, I=10Hz, 1H, austauschbar gegen D); 7,2-7,35 (m, 5H); 10,8 (s, 1H, austauschbar gegen D)

### Beispiele 6(1)-6(7):

### Herstellung verschiedener 1-Benzyl-5-alkoxy-imidazolin-2,4-dione ausgehend von isoliertem 1-Benzyl-5-hydroxy-imidazolin-2,4-dion unter Verwendung von Thionylchlorid und den entsprechenden Alkoholen

In einer Apparatur, wie sie in Beispiel 4 beschrieben ist, werden unter denselben Reaktionsbedingungen ausgehend aus 309 Gew.-Teilen 1-Benzyl-5-hydroxyimidazolin-2,4-dion aus Beispiel 2 und den entsprechenden Alkoholen folgende weitere 1-Benzyl-5-alkoxy-imidazolin-2,4-dione hergestellt:

### Beispiel 6(1)

### 1-Benzyl-5-methoxy-imidazolin-2,4-dion, C₁₁H₁₂N₂O₃.

Fp.: 116°C, MS: Molekularpeak 220, NMR (400MHz, DMSO D₆): 3,1 (s, 3H); 4,4 (dd, I₁=44Hz, I₂=11Hz, 2H); 5,05 (s, 1H); 7,25-7,40 (m, 5H); 11,15 (s, 1H, austauschbar gegen D)

### Beispiel 6(2)

### 1-Benzyl-5-propoxy-imidazolin-2,4-dion, C₁₃H₁₆N₂O₃.

Fp.: 72°C, MS: Molekularpeak 248, NMR (400MHz, DMSO D₆): 0,7-0,8 (t, 3H); 1,25-145 (m, 2H); 3,15-3,35 (m, 2H); 4,45 (dd, I₁=36Hz, I₂=12Hz, 2H); 5,1 (s, 1H); 7,25-7,40 (m, 5H); 11,15 (s, 1H, austauschbar gegen D)

### Beispiel 6(3)

### 1-Benzyl-5-isopropoxy-imidazolin-2,4-dion, C₁₃H₁₆N₂O₃·

Fp.: 61°C, MS: Molekularpeak 248, NMR (400MHz, DMSO D₆): 0,95-1,0 (d, 3H); 1,05-1,1 (d, 3H); 3,75-3,85 (m, 1H); 4,45 (dd, I₁=67Hz, I₂=17Hz, 2H); 5,0 (s, 1H); 7,25-7,40 (m, 5H); 11,03 (s, 1H, austauschbar gegen D)

### Beispiel 6(4)

### 1-Benzyl-5-butoxy-imidazolin-2,4-dion, C₁₄H₁₈N₂O₃.

Fp.: 76°C, MS: Molekularpeak 262, NMR (400MHz, DMSO D₆): 0,75-0,85 (t, 3H); 1,1-1,4 (m, 4H); 3,2-3,4 (m, 2H); 4,4 (dd, I₁=34Hz, I₂=17Hz, 2H); 5,1 (s, 1H); 7,24-7,40 (m, 5H); 11,15 (s, 1H, austauschbar gegen D)

### Beispiel 6(5)

### 1-Benzyl-5-isobutoxy-imidazolin-2,4-dion, C₁₄H₁₈N₂O₃.

Fp.: 87°C, MS: Molekularpeak 262, NMR (400MHz, DMSO D₆): 0,7-0,8 (t, 6H); 1,55-1,65 (m, 1H); 3,0-3,2 (m, 2H); 4,45 (dd, I₁=39Hz, I₂=17Hz, 2H); 5,1 (s, 1H); 7,25-7,40 (m, 5H); 11,1 (s, 1H, austauschbar gegen D)

### Beispiel 6(6)

### 1-Benzyl-5-octyloxy-imidazolin-2,4-dion, C₁₈H₂₆N₂O₃·

Fp.: 53°C, MS: Molekularpeak 318, NMR (400MHz, DMSO D₆): 0,8-0,9 (t, 3H); 1,1-1,35 (m, 12H); 3,2-3,4 (m, 2H); 4,45 (dd, I₁=28Hz, I₂=17Hz, 2H); 5,05 (s, 1H); 7,25-7,35 (m, 5H); 11,15 (s, 1H, austauschbar gegen D)

### Beispiel 6(7)

### 1-Benzyl-5-[(2-ethylhexyl)oxy]-imidazolin-2,4-dion, C₁₈H₂₆N₂O₃.

Fp.: 67-68°C, MS: Molekularpeak 302, NMR (400MHz, DMSO D₆): 0,7-0,85 (m, 6H); 1,1-1,25 (m, 9H); 3,1-3,25 (m, 2H);4,45 (dd, I₁=39Hz, I₂=17Hz, 2H); 5,1 (s, 1H); 7,25-7,40 (m, 5H); 11,15 (s, 1H, austauschbar gegen D)

### Beispiel 7

### Herstellung von 5-Ethoxy-1-(3-phenylpropyl)-imidazolin-2,4-dion

In Analogie zu Beispiel 4 wird durch Umsetzung von 5-Hydroxy-1-(3-phenylpropyl)-imidazolin-2,4-dion aus Beispiel 5(1) mit Thionylchlorid und Ethanol das **5-Ethoxy-1-(3-phenylpropyl)-imidazolin-2,4-dion** hergestellt.
Die Substanz C₁₄H₁₈N₂O₃ besitzt einen Schmelzpunkt Fp.: 64°C. MS: Molekularpeak 262, NMR (400MHz, DMSO D₆): 1,1-1,15 (t, 3H); 1,75-1,9 (m, 2H); 2,55-2,65 (t, 2H); 3,1-3,6 (m, 4H); 5,15 (s, 1H); 7,15-7,3 (m, 5H); 11,0 (s, 1H, austauschbar gegen D)

### Beispiel 8

### Herstellung von 5-Ethoxy-1-(2-phenylethyl)-imidazolin-2,4-dion

In Analogie zu Beispiel 4 wird durch Umsetzung von 5-Hydroxy-1-(2-phenylethyl)-imidazolin-2,4-dion aus Beispiel 5(7) mit Thionylchlorid und Ethanol das **5-Ethoxy-1-(2-phenylethyl)-imidazolin-2,4-dion** hergestellt.

Die Substanz C₁₃H₁₆N₂O₃ besitzt einen Schmelzpunkt Fp. von 120°C. MS: Molekularpeak 248, NMR (400MHz, DMSO D₆): 1,1-1,15 (t, 3H); 1,75-1,9 (m, 2H); 2,55-2,65 (t, 2H); 3,1-3,6 (m, 2H; 5,15 (s, 1H); 7,15-7,3 (m, 5H); 11,0 (s, 1H, austauschbar gegen D).

### Vergleichsbeispiel 9

### Umsetzung von N-Alkylharnstoffen mit Glyoxylsäure

In einer Apparatur, wie sie in Beispiel 2 beschrieben ist, wird versucht, unter den in Beispiel 2 beschriebenen Bedingungen durch Umsetzung von N-Alkylharnstoffen die entsprechenden 1-Alkyl-5-hydroxy-imidazolin-2,4-dione herzustellen.

Es werden zu 148 Gew.-Teilen 50%iger Glyoxylsäure, 100 Gew.-Teilen Wasser, 100 Gew.-Teilen Essigsäure bei 100°C im Verlauf von 1 Std. 74 Gew.-Teile N-Methylharnstoff in mehreren kleinen gleichen Portionen eingetragen. Das Reaktionsgemisch wird 1 Std. bei 100°C nachgerührt und anschließend wie in Beispiel 2 beschrieben aufgearbeitet. Es werden nur 37 Gew.-Teile eines schmierigen Kristallisates erhalten, das durch Kristallisation aus den gebräuchlichen Lösungsmitteln (Ethanol, Diethylether, Methylenchlorid, Toluol, Ethylacetat) nicht gereinigt werden kann. Die NMR-Analyse des Kristallisates zeigt eine Vielzahl von Substanzen, darunter auch die beiden isomeren Hydantoine: 1-Methyl-5-hydroxyimidazolin-2,4-dion mit dem charakteristischen NH-Signal bei 11,1 ppm und das isomere 3-Methyl-5-hydroxy-imidazolin-2,4-dion mit dem charakteristischen NMR-Signal bei 8,6 ppm. Die Ausbeute an beiden Hydantoinen wird anhand des NMR-Spektrums auf maximal jeweils 10% geschätzt.

Die Umsetzungen von N-Butylharnstoff, N-Octylharnstoff und N-Dodecylharnstoff mit Glyoxylsäure liefern ähnlich klebrige Produkte, aus denen keine einheitlichen Produkte isoliert werden konnten und deren NMR-Analytik neben den gewünschten 1-Alkyl-5-hydroxyimidazolin-2,4-dionen auch eine Vielzahl anderer Substanzen zeigten.

## Patentansprüche

1. Verfahren zur Herstellung von 1-substituierten 5-Hydroxy-imidazolin-2,4-dionen der Formel (I) 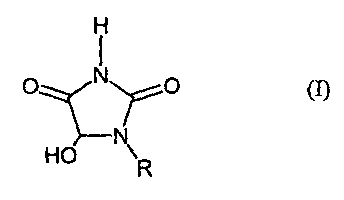 wobei R für einen gegebenenfalls substituierten C₇-C₁₈-Aralkylrest steht, durch Umsetzung von Glyoxylsäure mit N-substituiertem Harnstoff der Formel RNH-CO-NH₂, wobei R die zuvor genannte Bedeutung besitzt, **dadurch gekennzeichnet, dass** das Verfahren in einer 10-80 %igen wäßrigen Lösung und in Gegenwart eines sauren Katalysators durchgeführt wird.

2. Verfahren nach Anspruch 1, **dadurch gekennzeichnet, dass** N-substituierte Harnstoffe eingesetzt werden, bei denen R für einen gegebenenfalls substituierten C₇-C₁₂-Aralkylrest steht

3. Verfahren nach Anspruch 1 oder 2, **dadurch gekennzeichnet, dass** R für einen Benzylrest steht.

4. Verfahren nach einem oder mehreren der Ansprüche 1-3, **dadurch gekennzeichnet, dass** die Arylreste im Rest R durch 1, 2, 3, 4 oder 5 gleiche oder verschiedene Reste aus der Gruppe der Halogen-, bevorzugt Chlor, C₁-C₁₂-Alkyl-, bevorzugt Methyl, NO₂, C₁-C₁₂-Alkoxy-, bevorzugt Methoxy, und Phenoxyreste substituiert sind.

5. Verfahren nach einem oder mehreren der Ansprüche 1-4, **dadurch gekennzeichnet, dass** als saurer Katalysator eine Protonensäure eingesetzt wird,

6. Verfahren nach Anspruch 5, **dadurch gekennzeichnet, dass** Ameisensäure, Essigsäure, Propionsäure, Oxalsäure, Borsäure, Kalium- oder Natriumdihydrogenphosphat, Kalium- oder Natriumhydrogenphosphat, Phosphorsäure oder Alkalimetallhydrogensulfate eingesetzt werden.

7. Verfahren nach einem oder mehreren der Ansprüche 1-6, **dadurch gekennzeichnet, dass** das molare Verhältnis der Glyoxylsäure zum N-substituierten Harnstoff (0,5-5):1, bevorzugt (0,8-2):1 beträgt.

8. Verfahren nach einem oder mehreren der Ansprüche 1-7, **dadurch gekennzeichnet, dass** das 1-substituierte 5-Hydroxy-imidazolin-2,4-dion durch Kristallisation isoliert wird, wobei bevorzugt in Gegenwart eines organischen Lösungsmittels, insbesondere von Methylenchlorid, Chlorbenzol, aliphatischen Alkoholen oder Estern, gearbeitet wird.

9. Verfahren nach einem oder mehreren der Ansprüche 1-8, **dadurch gekennzeichnet, dass** das 1-substituierte 5-Hydroxy-imidazolin-2,4-dion der Formel I anschließend zum 1-substituierten 5-Alkoxy-imidazolin-2,4-dion der Formel II 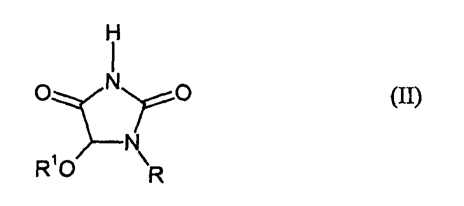 umgesetzt wird, wobei R die für Formel I genannte Bedeutung hat und R¹ für einen geradkettigen oder verzweigten C₁-C₁₈-Alkylrest, bevorzugt für Methyl, Ethyl, Propyl, iso-Propyl, Butyl, iso-Butyl, Octyl oder 2-Ethylhexyl und insbesondere für Ethyl steht.

10. Verfahren nach Anspruch 9, **dadurch gekennzeichnet, dass** das 1-substituierte 5-Hydroxy-imidazolin-2,4-dion der Formel I mit einem Alkohol der Formel R¹OH in Gegenwart eines sauren Katalysators zum 1-substituierten 5-Alkoxy-imidazolin-2,4-dion der Formel II umgesetzt wird.

11. Verfahren nach Anspruch 9, **dadurch gekennzeichnet, dass** das 1-substituierte 5-Hydroxy-imidazolin-2,4-dion der Formel I mit Orthoameisensäuretri-(C₁-C₁₈)-alkylester zum 1-substituierten 5-Alkoxy-imidazolin-2,4-dion der Formel II umgesetzt wird.

12. Verfahren nach Anspruch 9, **dadurch gekennzeichnet, dass** das 1-substituierte 5-Hydroxy-imidazolin-2,4-dion der Formel I zunächst mit Thionylchlorid und anschließend mit einem Alkohol R¹OH, wobei R¹ die zuvor genannte Bedeutung hat, zum 1-substituierten 5-Alkoxy-imidazolin-2,4-dion der Formel II umgesetzt wird.

## Claims

1. Process for preparing 1-substituted 5-hydroxy-imidazoline-2,4-diones of the formula (I) 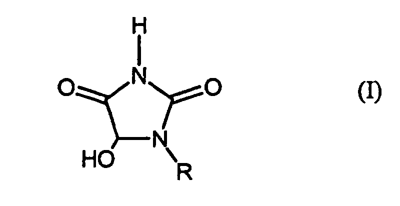 where R represents a substituted or unsubstituted C₇-C₁₈-aralkyl radical, by reacting glyoxylic acid with an N-substituted urea of the formula RNH-CO-NH₂, where R is as defined above, **characterized in that** the process is carried out in a 10-80% strength aqueous solution in the presence of an acid catalyst.

2. Process according to Claim 1, **characterized in that** the N-substituted ureas used are ones in which R represents a substituted or unsubstituted C₇-C₁₂- aralkyl radical.

3. Process according to Claim 1 or 2, **characterized in that** R represents a benzyl radical.

4. Process according to one or more of Claims 1-3, **characterized in that** the aryl radicals in the radical R are substituted by 1, 2, 3, 4 or 5 identical or different radicals selected from the group consisting of halogen, preferably chlorine, C₁-C₁₂-alkyl, preferably methyl, NO₂, C₁-C₁₂-alkoxy, preferably methoxy, and phenoxy.

5. Process according to one or more of Claims 1-4, **characterized in that** the acid catalyst used is a protic acid.

6. Process according to Claim 5, **characterized in that** formic acid, acetic acid, propionic acid, oxalic acid, boric acid, potassium or sodium dihydrogenphosphate, potassium or sodium hydrogenphosphate, phosphoric acid or alkali metal hydrogensulphates are used.

7. Process according to one or more of Claims 1-6, **characterized in that** the molar ratio of glyoxylic acid to the N-substituted urea is (0.5-5):1, preferably (0.8-2):1.

8. Process according to one or more of Claims 1-7, **characterized in that** the 1-substituted 5-hydroxy-imidazoline-2,4-dione is isolated by crystallization, preferably in the presence of an organic solvent, in particular methylene chloride, chlorobenzene, aliphatic alcohols or esters.

9. Process according to one or more of Claims 1-8, **characterized in that** the 1-substituted 5-hydroxy-imidazoline-2,4-dione of the formula I is subsequently converted into the 1-substituted 5-alkoxy-imidazoline-2,4-dione of the formula II 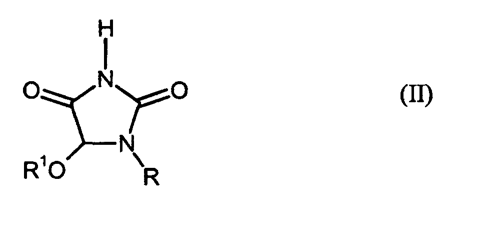 where R is as defined for formula I and R' represents a straight-chain or branched C₁-C₁₈-alkyl radical, preferably methyl, ethyl, propyl, iso-propyl, butyl, iso-butyl, octyl or 2-ethylhexyl and in particular ethyl.

10. Process according to Claim 9, **characterized in that** the 1-substituted 5-hydroxy-imidazoline-2,4-dione of the formula I is reacted with an alcohol of the formula R¹OH in the presence of an acid catalyst to form the 1-substituted 5-alkoxy-imidazoline-2,4-dione of the formula II.

11. Process according to Claim 9, **characterized in that** the 1-substituted 5-hydroxy-imidazoline-2,4-dione of the formula I is reacted with tri-(C₁-C₁₈)-alkyl orthoformate to form the 1-substituted 5-alkoxy-imidazoline-2,4-dione of the formula II.

12. Process according to Claim 9, **characterized in that** the 1-substituted 5-hydroxy-imidazoline-2,4-dione of the formula I is reacted firstly with thionyl chloride and subsequently with an alcohol R¹OH, where R¹ is as defined above, to form the 1-substituted 5-alkoxy-imidazoline-2,4-dione of the formula II.

## Revendications

1. Procédé de préparation de 5-hydroxyimidazoline-2,4-diones substituées en la position 1, de la formule (I) : 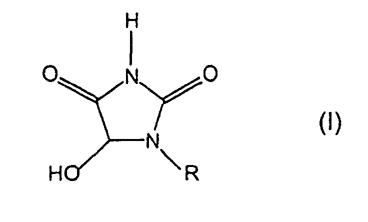 où R représente un reste aralcoyle en C₇-C₁₈ le cas échéant substitué, par réaction d'acide glyoxylique avec de l'urée N-substituée de la formule RNH-CO-NH₂, où R possède la signification indiquée ci-dessus, **caractérisé en ce que** le procédé est réalisé en solution aqueuse à 10-80% et en présence d'un catalyseur acide.

2. Procédé suivant la revendication 1, **caractérisé en ce que** l'on met en oeuvre une urée N-substituée, dans laquelle R représente un reste aralcoyle en C₇-C₁₈ le cas échéant substitué.

3. Procédé suivant la revendication 1 ou 2, **caractérisé en ce que** R représente un reste benzyle.

4. Procédé suivant l'une ou plusieurs des revendications 1 à 3, **caractérisé en ce que** le reste aryle dans le reste R est substitué par 1, 2, 3, 4 ou 5 restes identiques ou différents, du groupe des atomes d'halogène, de préférence l'atome de chlore, un radical alcoyle en C₁-C₁₂, de préférence le radical méthyle, le radical NO₂, un radical alcoxy en C₁-C₁₂, de préférence le radical méthoxy et le radical phényle.

5. Procédé suivant l'une ou plusieurs des revendications 1 à 4, **caractérisé en ce que** l'on met en oeuvre comme catalyseur acide, un acide protonique.

6. Procédé suivant la revendication 5, **caractérisé en ce que** l'on met en oeuvre l'acide formique, l'acide acétique, l'acide propionique, l'acide oxalique, l'acide borique, le dihydrogénophosphate de potassium ou de sodium, l'hydrogénophosphate de potassium ou de sodium, l'acide phosphorique ou un hydrogénosulfate de métal alcalin.

7. Procédé suivant l'une ou plusieurs des revendications 1 à 6, **caractérisé en ce que** le rapport molaire de l'acide glyoxylique à l'urée N-substituée se situe dans l'intervalle de (0,5-5):1, de préférence de (0,8-2):1.

8. Procédé suivant l'une ou plusieurs des revendications 1 à 7, **caractérisé en ce que** la 5-hydroxyimidazoline-2,4-dione substituée en la position 1 est isolée par cristallisation, où on travaille de préférence en présence d'un solvant organique, en particulier de chlorure de méthylène, de chlorobenzène, d'alcools ou d'esters aliphatiques.

9. Procédé suivant l'une ou plusieurs des revendications 1 à 8, **caractérisé en ce que** la 5-hydroxyimidazoline-2,4-dione substituée en la position 1 de la formule I est ensuite mise à réagir pour obtenir la 5-alcoxyimidazoline-2,4-dione substituée en la position 1 de la formule II : 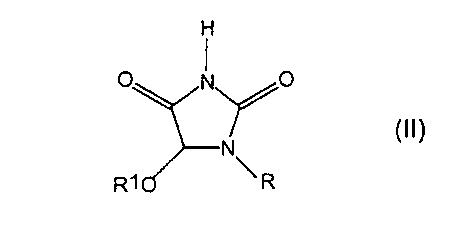 où R a la signification indiquée à la formule I et R¹ représente un reste alcoyle en C₁-C₁₈ linéaire ou ramifié, de préférence le radical méthyle, éthyle, propyle, isopropyle, butyle, isobutyle, octyle ou 2-éthylhexyle et en particulier, le radical éthyle.

10. Procédé suivant la revendication 9, **caractérisé en ce que** l'on fait réagir la 5-hydroxyimidazoline-2,4-dione substituée en la position 1 de la formule I avec un alcool de la formule R¹OH en présence d'un catalyseur acide pour obtenir la 5-alcoxyimidazoline-2,4-dione substituée en la position 1 de la formule II.

11. Procédé suivant la revendication 9, **caractérisé en ce que** l'on fait réagir la 5-hydroxyimidazoline-2,4-dione substituée en la position 1 de la formule I avec un ester alcoylique en C₁-C₁₈ de l'acide orthoformique pour obtenir la 5-alcoxyimidazoline-2,4-dione substituée en la position 1 de la formule II.

12. Procédé suivant la revendication 9, **caractérisé en ce que** l'on fait réagir la 5-hydroxyimidazoline-2,4-dione substituée en la position 1 de la formule I, d'abord avec le chlorure de thionyle et ensuite, avec un alcool R¹OH, où R¹ a la signification indiquée ci-dessus, pour obtenir la 5-alcoxyimidazoline-2,4-dione substituée en la position 1 de la formule II.
